# EUROPEAN PATENT APPLICATION

(11) **EP 4 804 495 A1**
(43) Date of publication of application: **09.09.2026**
(21) Application number: 26162602.2
(22) Date of filing: 05.03.2026
(51) Int. Cl.: H04L 67/025, A61B 1/24, G06F 16/182, G16H 10/60, G16H 30/20, G16H 40/20, G16H 40/67, H04L 67/10, H04L 67/12

(54) **DENTAL HYBRID CLOUD SYSTEM AND METHOD OF DRIVING THE SAME**

(30) Priority: 07.03.2025 KR 20250029675; 01.04.2025 WO PCT/KR2025/004251
(71) Applicant: Imagoworks Inc., Seoul 06034 (KR)
(72) Inventor: YU, Jaeil, 06115 Seoul (KR); JUNG, Daeil, 14687 Bucheon-si Gyeonggi-do (KR)
(74) Representative: BCKIP Part mbB

(57) **Abstract**

A dental hybrid cloud system includes a mode controller and a dental service provider. The mode controller outputs a first mode control signal and a second mode control signal. The dental service provider operates in a first mode for providing a digital dental service using an on-premise in response to the first mode control signal, and operates in a second mode for providing the digital dental service the on-premise and in a cloud in response to the second mode control signal.

## Description

This application claims priority to Korean Patent Application No. 10-2025-0029675, filed on March 7, 2025 in the Korean Intellectual Property Office (KIPO) and International Patent Application No. PCT/KR2025/004251 filed on April 1, 2025.

### BACKGROUND

### 1. Technical Field

Embodiments relate to a dental hybrid cloud system and a method of driving the same. More particularly, embodiments relate to a dental hybrid cloud system for providing a digital dental service which handles sensitive medical data and a method of driving the same.

### 2. Description of the Related Art

An on-premise refers to a method in which an enterprise or an individual owns and operates IT infrastructure, such as a server, a storage, and a network, in their own physical facility. A cloud refers to a method in which this IT infrastructure is provided through an internet.

The on-premise offers superior data security and control because the IT infrastructure is directly managed, but an initial setup and a maintenance are complex and expensive. On the other hand, the cloud flexibly provides computer resources to offer a user cost-effective and scalable solution, but raises concerns in terms of the data security and the control.

A digital dental service may include medical data of the user, and the medical data of the user may be sensitive. Therefore, when providing the digital dental service to the user, both cost and security aspects need to be considered.

### SUMMARY

Embodiments provide a dental hybrid cloud system for providing a digital dental service in consideration of cost and security aspects.

Embodiments provide a method of driving the dental hybrid cloud system.

In an example dental hybrid cloud system according to the present inventive concept, the dental hybrid cloud system includes a mode controller configured to output a first mode control signal and a second mode control signal, and a dental service provider configured to operate in a first mode for providing a digital dental service using an on-premise in response to the first mode control signal, and to operate in a second mode for providing the digital dental service the on-premise and in a cloud in response to the second mode control signal.

In an embodiment, medical data of a user and system driving data other than the medical data may be stored in the on-premise when the dental service provider operates in the first mode.

In an embodiment, medical data of a user may be stored in the on-premise, and system driving data other than the medical data may be stored in the on-premise and in the cloud or stored in the cloud when the dental service provider operates in the second mode.

In an embodiment, among the system driving data, system driving data requiring a high-performance computation may be stored in the cloud, and among the system driving data, system driving data requiring a general performance computation rather than the high-performance computation may be stored in the on-premise when the system driving data is stored in the on-premise and the cloud.

In an embodiment, the system driving data stored in the on-premise and the system driving data stored in the cloud may be synchronized when the system driving data is stored in the on-premise and in the cloud.

In an embodiment, the on-premise and the cloud may be connected through a dedicated line using a virtual private network (VPN) when the dental service provider operates in the second mode.

In an embodiment, the digital dental service may be provided using a user terminal when the dental service provider operates in the second mode.

In an embodiment, the user terminal may be a desktop computer, a laptop computer, a tablet computer, or a smartphone, and the digital dental service may be provided through a web browser or an application using the user terminal.

In an embodiment, the user terminal may be previously registered for the digital dental service.

In an embodiment, the dental service provider may be configured to generate a data access log including an information of a terminal accessing the digital dental service. The dental service provider may be configured to generate an abnormal access notification when a terminal not previously registered accesses the digital dental service.

In an embodiment, the mode controller may be configured to output the first mode control signal or the second mode control signal according to a selection of a user.

In an embodiment, the mode controller may be configured to output the first mode control signal or the second mode control signal according to a number of users or a data throughput.

In an example method of driving a dental hybrid cloud system, the method includes outputting a first mode control signal and a second mode control signal, and operating in a first mode for providing a digital dental service using an on-premise in response to the first mode control signal, and operating in a second mode for providing the digital dental service using the on-premise and a cloud in response to the second mode control signal.

In an embodiment, medical data of a user and system driving data other than the medical data may be stored in the on-premise when the dental hybrid cloud system operates in the first mode.

In an embodiment, medical data of a user may be stored in the on-premise, and system driving data other than the medical data may be stored in the on-premise and in the cloud or stored in the cloud when the dental hybrid cloud system operates in the second mode.

In an embodiment, among the system driving data, system driving data requiring a high-performance computation may be stored in the cloud, and among the system driving data, system driving data requiring a general performance computation rather than the high-performance computation may be stored in the on-premise when the system driving data is stored in the on-premise and the cloud.

In an embodiment, the system driving data stored in the on-premise and the system driving data stored in the cloud may be synchronized when the system driving data is stored in the on-premise and in the cloud.

In an embodiment, the on-premise and the cloud may be connected through a dedicated line using a virtual private network (VPN) when the dental hybrid cloud system operates in the second mode.

In an embodiment, the digital dental service may be provided using a user terminal when the dental hybrid cloud system operates in the second mode.

In an embodiment, the user terminal may be a desktop computer, a laptop computer, a tablet computer, or a smartphone, and the digital dental service may be provided through a web browser or an application using the user terminal.

According to the dental hybrid cloud system and the method, the dental hybrid cloud system may operate in the first mode for using only the on-premise, and in the second mode for using both the on-premise and the cloud. Accordingly, the dental hybrid cloud system may provide the digital dental service in consideration of the security of the on-premises and the flexibility of the cloud.

### BRIEF DESCRIPTION OF THE DRAWINGS

The above and other features and advantages of the present inventive concept will become more apparent by describing in detailed embodiments thereof with reference to the accompanying drawings, in which:
FIG. 1 is a block diagram illustrating a dental hybrid cloud system according to embodiments of the present inventive concept; and
FIG. 2 is a flowchart illustrating a method of driving a dental hybrid cloud system according to embodiments of the present inventive concept.

### DETAILED DESCRIPTION OF THE EMBODIMENTS

The present inventive concept now will be described more fully hereinafter with reference to the accompanying drawings, in which embodiments of the present inventive concept are shown. The present inventive concept may, however, be embodied in many different forms and should not be construed as limited to the embodiments set forth herein.

Rather, these embodiments are provided so that this disclosure will be thorough and complete, and will fully convey the scope of the present inventive concept to those skilled in the art. Like reference numerals refer to like elements throughout.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present inventive concept.

The terminology used herein is for the purpose of describing particular embodiments only and is not intended to be limiting of the present inventive concept. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

All methods described herein can be performed in a suitable order unless otherwise indicated herein or otherwise clearly contradicted by context. The use of any and all examples, or exemplary language (e.g., "such as"), is intended merely to better illustrate the invention and does not pose a limitation on the scope of the invention unless otherwise claimed. No language in the specification should be construed as indicating any non-claimed element as essential to the practice of the inventive concept as used herein.

Hereinafter, the present inventive concept will be explained in detail with reference to the accompanying drawings.

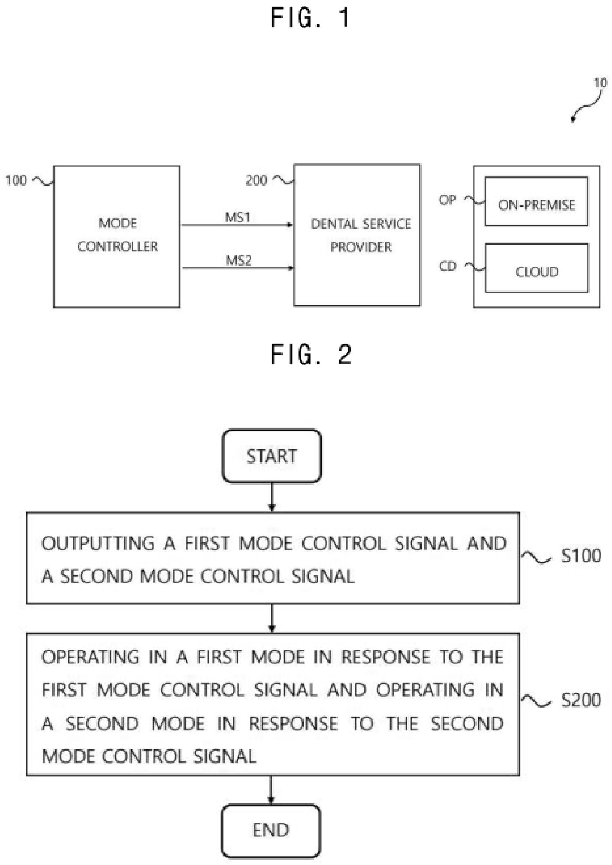

FIG. 1 is a block diagram illustrating a dental hybrid cloud system 10 according to embodiments of the present inventive concept.

Referring to FIG. 1, the dental hybrid cloud system 10 provides a digital dental service by considering a security of an on-premise OP environment and a flexibility of a cloud CD. Here, the cloud CD may be a public cloud. A storage of the on-premise OP may be NAS(Network Attached Storage), SAN(Storage Area Network), etc., and a storage of the cloud CD storage may be an Azure storage, an AWS bucket, etc. The digital dental service refers to providing a precise and efficient service using a digital technology during dental treatment and care. For example, the digital dental service may be referred to as a dentbird solution. The dentbird solution is a system configured with a microservice architecture using a container technology and Kubernetes based on OCI(Open Container Initiative). The dentbird solution may include dental CAD functions and AI-based diagnosis support functions.

The dental hybrid cloud system 10 may include a mode controller 100 and a dental service provider 200.

The mode controller 100 may output a first mode control signal MS1 and a second mode control signal MS2. The first mode control signal MS1 and the second mode control signal MS2 may be signals for controlling modes of the dental hybrid cloud system 10. In an embodiment, the mode controller 100 may output the first mode control signal MS1 or the second mode control signal MS2 based on a selection of a user. In another embodiment, the first mode control signal MS1 or the second mode control signal MS2 may be output based on a number of users or a data throughput.

The dental service provider 200 may operate in a first mode for providing the digital dental service using the on-premise OP, in response to the first mode control signal MS1, and in a second mode for providing the digital dental service using the on-premise OP and the cloud CD, in response to the second mode control signal MS2.

Data of the dental hybrid cloud system 10 may include medical data of a user, and the medical data may be sensitive data. The on-premise OP refers to a method in which an enterprise or an individual owns and operates IT infrastructure, such as a server, a storage, and a network, in their own physical facility. Since the on-premise OP provides the digital dental service without an internet, the on-premise OP may be advantageous in terms of a security and may be suitable for storing and processing the medical data. In addition, the on-premise OP may minimize a network latency. On the other hand, the cloud CD refers to a method in which the IT infrastructure is provided through the internet. Therefore, the cloud CD may be cost-effective and suitable for storing and processing system driving data rather than medical data.

In the first mode, the digital dental service may be provided using only the on-premise OP. The medical data and system driving data may be stored in the on-premise OP.

In the first mode, the dental hybrid cloud system 10 may require a significant security. Therefore, the dental hybrid cloud system 10 may be updated collectively for all update configurations or selectively for some of the update configurations. In addition, to enable an efficient update even in a limited environment, the dental hybrid cloud system 10 may be differentially updated for the update configurations.

In the second mode, the digital dental service may be provided using the on-premise OP and the cloud CD. In an embodiment, the medical data may be stored in the on-premise OP, and the system driving data may be stored in the cloud CD. In another embodiment, the medical data may be stored in the on-premise OP, and the system driving data may be stored in the on-premise OP and the cloud CD. When the system driving data is stored in the on-premise OP and the cloud CD, among the system driving data, system driving data requiring a high-performance computation may be stored in the cloud CD, and among the system driving data, system driving data requiring general performance computation rather than high-performance computation may be stored in the on-premise OP. When the system driving data is stored in the on-premise OP and the cloud CD, the system driving data stored in the on-premise OP and the system driving data stored in the cloud CD may be synchronized.

In the second mode, the on-premise OP and the cloud CD may be connected through a dedicated line using a Virtual Private Network(VPN). Specifically, the virtual private network encrypts data using the dedicated line, such that the on-premise OP and the cloud CD may be stably connected.

In the second mode, the digital dental service may be provided using a user terminal. In an embodiment, the user terminal may be a device such as a desktop computer, a laptop computer, a tablet computer, a wearable device, or a smartphone, and the digital dental service may be provided through a web browser or application using the user terminal. On the other hand, to enhance a security, in an embodiment, the user terminal should be previously registered before the digital dental service is provided to the user terminal. In addition, the dental service provider 200 may generate a data access log including an information of a terminal accessing the digital dental service. Therefore, when a terminal, which is not previously registered, accesses the digital dental service, the dental service provider 200 may generate an abnormal access notification to inform the user of an abnormal access to the digital dental service.

In the second mode, since the dental service provider 200 uses the cloud CD and the cloud CD uses the internet, the dental hybrid cloud system 10 may be quickly updated. Therefore, when a security update is required for the dental hybrid cloud system 10, the dental hybrid cloud system 10 may be quickly updated.

In the second mode, the dental service provider 200 may quickly provide static assets using CDN(Content Delivery Network) and improve a response speed with an optimized code.

As such, the dental hybrid cloud system 10 may operate in the first mode for using only the on-premise OP, and operate in the second mode for using both the on-premise OP and the cloud CD. Accordingly, the dental hybrid cloud system 10 may provide the digital dental service by considering the security of the on-premise OP and the flexibility of the cloud CD. Here, the dental hybrid cloud system 10 may use AWS SPP(Solution Provider Program), Azure MSP(Managed Service Provider), AWS CF(CloudFormation), Architecture Provisioning such as Azure Resource Manager Templates, and Cloud SDK in order to use both the on-premise OP and the cloud CD.

FIG. 2 is a flowchart illustrating a method of driving a dental hybrid cloud system 10 according to embodiments of the present inventive concept.

Referring to FIGS. 1 and 2, a method of driving a dental hybrid cloud system 10 may include outputting a first mode control signal MS1 and a second mode control signal MS2 (step S100), and operating in a first mode for providing a digital dental service using an on-premise OP in response to the first mode control signal MS1 and a second mode for providing the digital dental service using the on-premise OP and a cloud CD in response to the second mode control signal MS2.

The method of driving the dental hybrid cloud system 10 of FIG. 2 is substantially equal to the dental hybrid cloud system 10 of FIG. 1. Therefore, redundant descriptions will be omitted.

As such, the method of driving the dental hybrid cloud system 10 may operate in the first mode for using only the on-premise OP, and operate in the second mode for using both the on-premise OP and the cloud CD. Accordingly, the method of driving the dental hybrid cloud system 10 may provide the digital dental service by considering the security of the on-premise OP and the flexibility of the cloud CD.

According to an embodiment of the present inventive concept, a non-transitory computer-readable storage medium having stored thereon program instructions of the method of driving the dental hybrid cloud system 10 according to an embodiment of the present inventive concept may be provided. The above mentioned method may be written as a program executed on the computer. The method may be implemented in a general purpose digital computer which operates the program using a computer-readable medium. In addition, the structure of the data used in the above mentioned method may be written on a computer readable medium through various means. The computer readable medium may include program instructions, data files and data structures alone or in combination. The program instructions written on the medium may be specially designed and configured for the present inventive concept, or may be generally known to a person skilled in the computer software field. For example, the computer readable medium may include a magnetic medium such as a hard disk, a floppy disk and a magnetic tape, an optical recording medium such as CD-ROM and DVD, a magneto-optical medium such as floptic disc and a hardware device specially configured to store and execute the program instructions such as ROM, RAM and a flash memory. For example, the program instructions may include a machine language codes produced by a compiler and high-level language codes which may be executed by a computer using an interpreter or the like. The hardware device may be configured to operate as one or more software modules to perform the operations of the present inventive concept.

In addition, the above mentioned method of driving the dental hybrid cloud system 10 may be implemented in a form of a computer-executed computer program or an application which are stored in a storage method.

The present inventive concept relates to a dental hybrid cloud system and a method of driving the same, which may reduce an effort and a time required for an computational processing operation and improve an accuracy and a productivity.

The foregoing is illustrative of the present inventive concept and is not to be construed as limiting thereof. Although a few embodiments of the present inventive concept have been described, those skilled in the art will readily appreciate that many modifications are possible in the embodiments without materially departing from the novel teachings and advantages of the present inventive concept. Accordingly, all such modifications are intended to be included within the scope of the present inventive concept as defined in the claims. In the claims, means-plus-function clauses are intended to cover the structures described herein as performing the recited function and not only structural equivalents but also equivalent structures. Therefore, it is to be understood that the foregoing is illustrative of the present inventive concept and is not to be construed as limited to the specific embodiments disclosed, and that modifications to the disclosed embodiments, as well as other embodiments, are intended to be included within the scope of the appended claims. The present inventive concept is defined by the following claims, with equivalents of the claims to be included therein.

## Claims

1. A dental hybrid cloud system comprising:
a mode controller configured to output a first mode control signal and a second mode control signal; and
a dental service provider configured to operate in a first mode for providing a digital dental service using an on-premise in response to the first mode control signal, and to operate in a second mode for providing the digital dental service the on-premise and in a cloud in response to the second mode control signal.

2. The dental hybrid cloud system of claim 1, wherein medical data of a user and system driving data other than the medical data are stored in the on-premise when the dental service provider operates in the first mode.

3. The dental hybrid cloud system of claim 1, wherein medical data of a user is stored in the on-premise, and system driving data other than the medical data is stored in the on-premise and in the cloud or stored in the cloud when the dental service provider operates in the second mode.

4. The dental hybrid cloud system of claim 3, wherein among the system driving data, system driving data requiring a high-performance computation is stored in the cloud, and among the system driving data, system driving data requiring a general performance computation rather than the high-performance computation is stored in the on-premise when the system driving data is stored in the on-premise and the cloud.

5. The dental hybrid cloud system of claim 3, wherein the system driving data stored in the on-premise and the system driving data stored in the cloud are synchronized when the system driving data is stored in the on-premise and in the cloud.

6. The dental hybrid cloud system of claim 1, wherein the on-premise and the cloud are connected through a dedicated line using a virtual private network (VPN) when the dental service provider operates in the second mode.

7. The dental hybrid cloud system of claim 1, wherein the digital dental service is provided using a user terminal when the dental service provider operates in the second mode.

8. The dental hybrid cloud system of claim 7, wherein the user terminal is a desktop computer, a laptop computer, a tablet computer, or a smartphone, and the digital dental service is provided through a web browser or an application using the user terminal.

9. The dental hybrid cloud system of claim 7, wherein the user terminal is previously registered for the digital dental service.

10. The dental hybrid cloud system of claim 9, wherein the dental service provider is configured to generate a data access log including an information of a terminal accessing the digital dental service, and
wherein the dental service provider is configured to generate an abnormal access notification when a terminal not previously registered accesses the digital dental service.

11. The dental hybrid cloud system of claim 1, wherein the mode controller is configured to output the first mode control signal or the second mode control signal according to a selection of a user.

12. The dental hybrid cloud system of claim 1, wherein the mode controller is configured to output the first mode control signal or the second mode control signal according to a number of users or a data throughput.

13. A method for driving a dental hybrid cloud system, the method comprising:
outputting a first mode control signal and a second mode control signal; and
operating in a first mode for providing a digital dental service using an on-premise in response to the first mode control signal, and operating in a second mode for providing the digital dental service using the on-premise and a cloud in response to the second mode control signal.

14. The method of claim 13, wherein medical data of a user and system driving data other than the medical data are stored in the on-premise when the dental hybrid cloud system operates in the first mode.

15. The method of claim 13, wherein medical data of a user is stored in the on-premise, and system driving data other than the medical data is stored in the on-premise and in the cloud or stored in the cloud when the dental hybrid cloud system operates in the second mode.

16. The method of claim 15, wherein among the system driving data, system driving data requiring a high-performance computation is stored in the cloud, and among the system driving data, system driving data requiring a general performance computation rather than the high-performance computation is stored in the on-premise when the system driving data is stored in the on-premise and the cloud.

17. The method of claim 15, wherein the system driving data stored in the on-premise and the system driving data stored in the cloud are synchronized when the system driving data is stored in the on-premise and in the cloud.

18. The method of claim 13, wherein the on-premise and the cloud are connected through a dedicated line using a virtual private network (VPN) when the dental hybrid cloud system operates in the second mode.

19. The method of claim 13, wherein the digital dental service is provided using a user terminal when the dental hybrid cloud system operates in the second mode.

20. The method of claim 19, wherein the user terminal is a desktop computer, a laptop computer, a tablet computer, or a smartphone, and the digital dental service is provided through a web browser or an application using the user terminal.

## Amended claims

### Amended claims in accordance with Rule 137(2) EPC.

1. A dental hybrid cloud system comprising:
a mode controller configured to output a first mode control signal and a second mode control signal; and
a dental service provider configured to operate in a first mode for providing a digital dental service using only an on-premise IT infrastructure in response to the first mode control signal, and to operate in a second mode for providing the digital dental service in the on-premise and in a cloud in response to the second mode control signal;
wherein sensitive medical data of a user is stored in the on-premise, and system driving data other than the medical data is stored in the on-premise and in the cloud when the dental service provider operates in the second mode,
wherein the medical data of the user and the system driving data other than the medical data are stored in the on-premise when the dental service provider operates in the first mode, and
wherein among the system driving data, system driving data requiring a high-performance computation is stored in the cloud, and among the system driving data, system driving data requiring a general performance computation rather than the high-performance computation is stored in the on-premise when the system driving data is stored in the on-premise and the cloud.

2. The dental hybrid cloud system of claim 1, wherein the system driving data stored in the on-premise and the system driving data stored in the cloud are synchronized when the system driving data is stored in the on-premise and in the cloud.

3. The dental hybrid cloud system of claim 1, wherein the on-premise and the cloud are connected through a dedicated line using a virtual private network (VPN) when the dental service provider operates in the second mode.

4. The dental hybrid cloud system of claim 1, wherein the digital dental service is provided using a user terminal when the dental service provider operates in the second mode.

5. The dental hybrid cloud system of claim 4, wherein the user terminal is a desktop computer, a laptop computer, a tablet computer, or a smartphone, and the digital dental service is provided through a web browser or an application using the user terminal.

6. The dental hybrid cloud system of claim 4, wherein the user terminal is previously registered for the digital dental service; optionally,
wherein the dental service provider is configured to generate a data access log including an information of a terminal accessing the digital dental service, and
wherein the dental service provider is configured to generate an abnormal access notification when a terminal not previously registered accesses the digital dental service.

7. The dental hybrid cloud system of claim 1, wherein the mode controller is configured to output the first mode control signal or the second mode control signal according to a selection of a user; or
wherein the mode controller is configured to output the first mode control signal or the second mode control signal according to a number of users or a data throughput.

8. A method for driving a dental hybrid cloud system, the method comprising:
outputting a first mode control signal and a second mode control signal; and
operating in a first mode for providing a digital dental service using only an on-premise IT infrastructure in response to the first mode control signal, and operating in a second mode for providing the digital dental service using the on-premise and a cloud in response to the second mode control signal;
wherein sensitive medical data of a user is stored in the on-premise, and system driving data other than the medical data is stored in the on-premise and in the cloud when the dental hybrid cloud system operates in the second mode,
wherein the medical data of a user and the system driving data other than the medical data are stored in the on-premise when the dental hybrid cloud system operates in the first mode, and
wherein among the system driving data, system driving data requiring a high-performance computation is stored in the cloud, and among the system driving data, system driving data requiring a general performance computation rather than the high-performance computation is stored in the on-premise when the system driving data is stored in the on-premise and the cloud.

9. The method of claim 8, wherein the system driving data stored in the on-premise and the system driving data stored in the cloud are synchronized when the system driving data is stored in the on-premise and in the cloud.

10. The method of claim 8, wherein the on-premise and the cloud are connected through a dedicated line using a virtual private network (VPN) when the dental hybrid cloud system operates in the second mode.

11. The method of claim 8, wherein the digital dental service is provided using a user terminal when the dental hybrid cloud system operates in the second mode; optionally,
wherein the user terminal is a desktop computer, a laptop computer, a tablet computer, or a smartphone, and the digital dental service is provided through a web browser or an application using the user terminal.
